# EUROPEAN PATENT SPECIFICATION

(11) **EP 4 319 853 B1**
(45) Date of publication and mention of the grant of the patent: **30.10.2024**
(21) Application number: 22785791.9
(22) Date of filing: 15.09.2022
(51) Int. Cl.: A61M 25/04, A61M 25/09, A61M 25/01, A61M 25/00, A61B 17/00, A61F 2/24

(54) **AN ADJUSTABLE DEVICE FOR INSERTING A GUIDE WIRE INTO A BLOOD VESSEL**
EINE EINSTELLBARE VORRICHTUNG ZUM EINFÜHREN EINES FÜHRUNGSDRAHTES IN EIN BLUTGEFÄSS
UN DISPOSITIF RÉGLABLE POUR INSÉRER UN FIL DE GUIDAGE DANS UN VAISSEAU SANGUIN

(30) Priority: 20.09.2021 IT 202100024056
(43) Date of publication of application: 14.02.2024
(73) Proprietor: Vivheart S.r.l., 20121 Milano MI (IT)
(72) Inventor: MONTORFANO, Matteo, 20122 MILANO MI (IT)
(74) Representative: Barbaro, Gaetano
(86) International application number: PCT/IB2022/058701
(87) International publication number: WO 2023/042111

(56) References cited:
- EP-A1- 3 658 215
- US-A1- 2005 234 425

## Description

### TECHNICAL FIELD

The present disclosure relates in general to catheters and more in particular to a device for inserting a guidewire into a blood vessel, wherein the device is self-centering and adjustable, and may be inserted into an artery for directing a guidewire through a heart valve.

### BACKGROUND OF THE INVENTION

Percutaneous valve replacement (Transcatheter Aortic Valve Replacement or more briefly TAVR) is a technique performed by inserting a catheter containing a guidewire, which is used as a guide for the implantation of a cardiac valve prosthesis. The catheter is threaded through the femoral artery and pushed up to the proximity of the defective heart valve or through the chest, for exiting the guidewire contained in the catheter near the cardiac valve so that it passes throughout it. Once the guidewire has passed throughout the heart valve, it is used to guide a valve prosthesis to the heart, where it must be positioned. A difficulty related to this type of technique consists in the fact that the operator must be particularly skilled in order to quickly insert the guidewire through the faulty valve.

The imaging equipment currently used in operating rooms only allow a two-dimensional view and not a three-dimensional view of the guidewire approaching the defective heart valve. As a consequence, from the two-dimensional image the operator may have the wrong impression of correctly directing the guidewire to the center of the heart valve, which is also narrow and degenerated, crooked, when instead the apical end of the guidewire is going to abut against the walls of the aorta.

In any case, even if a second apparatus for images arranged so as to provide a different view were available, there would be the problem of positioning and properly orienting a catheter while pushing forward the guidewire. Moreover, the guidewire is made of yielding material so as not to damage the tissues with which it comes into contact, so it bends easily even when it should remain straight to pass through the defective valve.

This problem is even more pronounced in the so-called Valve-In-Valve technique (or more briefly VIV), in which a defective valve prosthesis is not completely removed, but is used as a frame in which a new percutaneous heart valve prosthesis is anchored. This new technique prevents the patient from the trauma of removing the old prosthetic valve, which remains attached to the heart tissue while the new valve prosthesis is attached to the old. Nevertheless this is critical because the guidewire, if pushed against the damaged portions of the old heart valve prosthesis, may cause the detachment of a part of it.

The document US2014/0207179 discloses a device, shown in Figure 1, for centering a catheter 190 in which to pass a surgical guide wire 192. This anterior device has a plurality of thread-like elements 186 of equal length having ends fixed to a distal element 188 and to a proximal element 194. The thread-like elements 186, having thermal memory, bend and move radially away over the internal catheter 190 the distal element 188 retracting it. If the device is inserted in a substantially cylindrical portion of a blood vessel, the thread-like elements 186 abut against the blood vessel wall in a practically symmetrical manner, so that the internal catheter 190 is substantially placed at the center of the blood vessel.

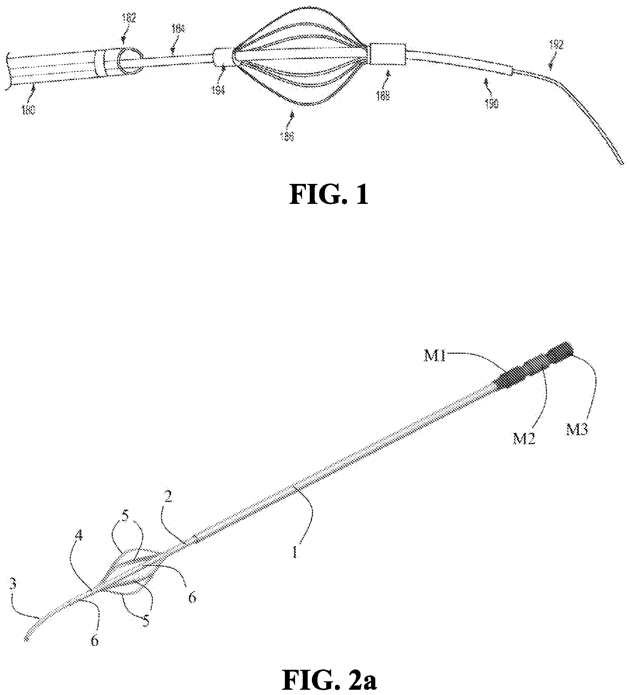

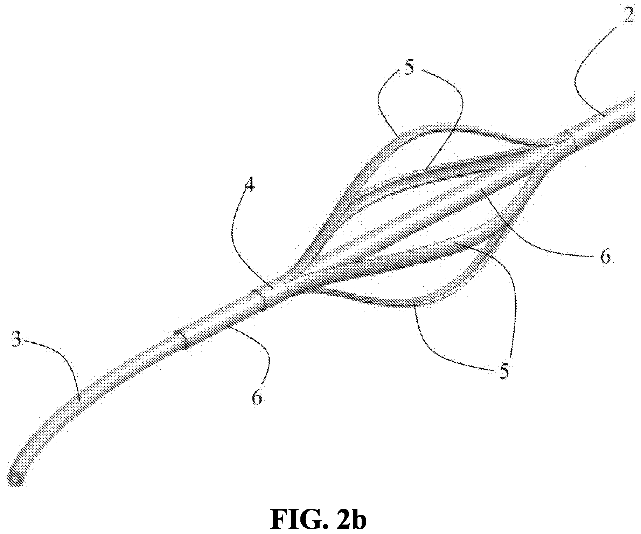

The document WO2019138334 discloses a device, shown in figure 2a and in the detail view of figure 2b, comprising three catheters 1, 2, 3, with different characteristics, one inserted inside the other, to direct a guiding wire through a blood vessel and / or heart valve. Like the device of figure 1, it has thread-like elements 5 of equal length having ends fixed to a distal element 4 and to a proximal element 2. The distal element slides over a tubular portion 6 of the intermediate catheter 2. A more internal catheter 3, intended to conduct a surgical guide wire, tends to spontaneously bend in a more accentuated manner the more it protrudes from the tubular portion 6 of the intermediate catheter 2. An outermost catheter 1 is arranged so that the intermediate catheter 2 and the more internal 3 can be contained therein, so that the thread-like elements 5 can expand freely, exiting the outermost catheter 1 in proximity to the point where a surgical intervention is to be performed.A drawback of this type of device is that, when inserted into a blood vessel and opened, the thread-like elements 186, 5 tend to flex along the circumferential direction as soon as they touch the blood vessel walls and move along the circumferential direction. This unexpected effect occurs in particular in large blood vessels such as the aorta. Consequently, often the catheter 190, 6, over which the distal element 188, 4 slides, is not immediately positioned in the desired way, for example in correspondence with the heart valves, so that the surgeon can only retract the thread-like elements 186, 5 into the external catheter 180, 1, and let them come out again until they are positioned in the desired way.

Document US2005/234425, on the basis of which the preamble of claim 1 has been formulated, discloses a catheter with an anchoring element.

### SUMMARY

An object of the present disclosure is to provide an adjustable device for inserting a guide wire suitable for heart valve prostheses into a blood vessel, defined in the attached claim 1, which solves at least partially the aforementioned drawbacks. The device allows the exit opening of the catheter into which the guide wire is inserted to be bent as desired, maintaining a firm coupling against the walls of the blood vessel in which it is inserted.

Tests carried out by the applicant with different prototypes have shown that it is easier to counteract unwanted displacements of the thread-like elements if the distal portion, to which the thread-like elements are connected, is fixed - for example by welding or gluing or other valid joining method - to the internal catheter and the internal catheter has a handle portion which allows the surgeon, by pulling it, to modulate the folding of the thread-like elements, around the longitudinal axis of the catheter. Furthermore, thanks to the fact that it is possible to shorten the distal portion of the external catheter with respect to the longitudinal axis, by withdrawing the internal catheter, it has been noted that it is particularly convenient to make the thread-like elements by means of a first plurality of notches and a second plurality of staggered notches between them along the longitudinal direction, carried out on a median portion of the external catheter, in order to quickly and firmly position the thread-like elements against irregular walls of a blood vessel.

Preferred embodiments are defined in the dependent claims.

### BRIEF DESCRIPTION OF THE DRAWINGS

Figure 1 shows a prior device for positioning a catheter carrying a surgical guide wire.
Figure 2a is an overall view of another prior device for positioning a catheter carrying a surgical guide wire.
Figure 2b is a detail view of the prior device of Figure 2a.
Figure 3 shows profile, top and detail views of an exemplary embodiment of this disclosure of a guide wire insertion device suitable for valve prostheses, in an extended configuration.
Figure 4 shows profile, top and detail views of the device of Figure 3 in an expanded configuration.
Figures 5a and 5b show in sequence how the thread-like elements fold / unfold by pulling / pushing a handle portion of the internal catheter of the device of Figures 3 and 4.
Figures 6a and 6b show sectional views of another exemplary embodiment of a device according to the present disclosure, with thread-like elements defined by staggered notches of different lengths.
Figures 7a to 7c show in perspective the device of Figures 6a and 6b with folded thread-like elements.

### DETAILED DESCRIPTION OF EXAMPLARY EMBODIMENTS

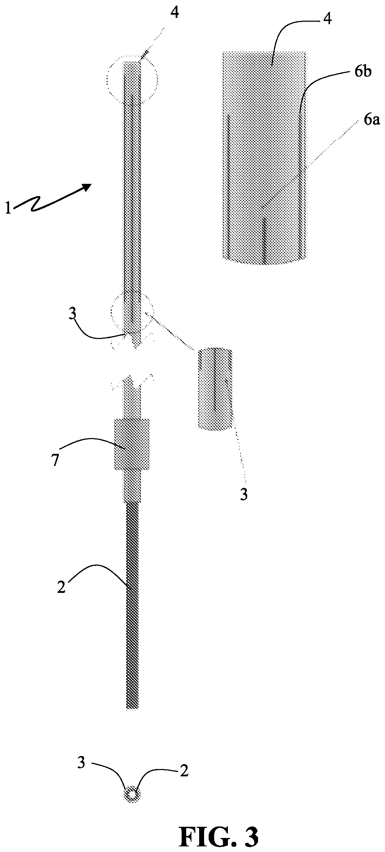

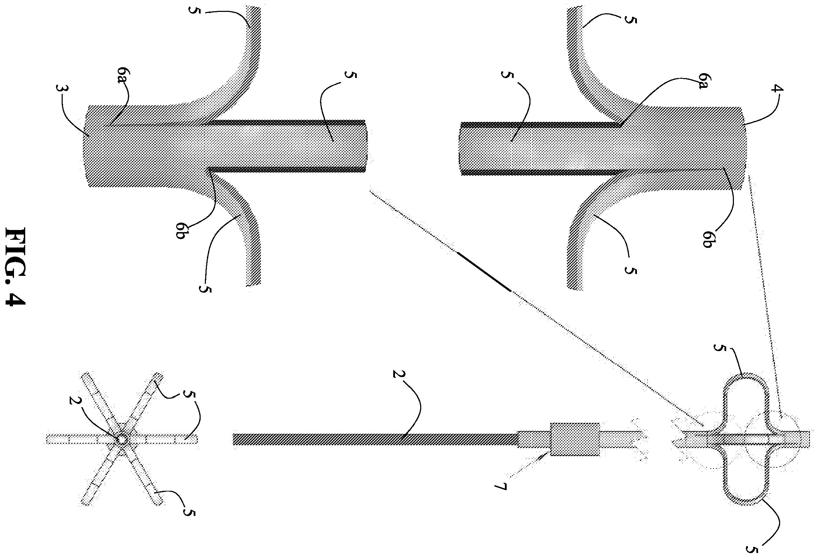

Characteristic features of the device of the present disclosure will be illustrated for simplicity with reference to the embodiment illustrated in Figures 3 and 4, but the skilled technician will recognize that the same observations hold, *mutatis mutandis,* for all the embodiments defined by the attached claims.

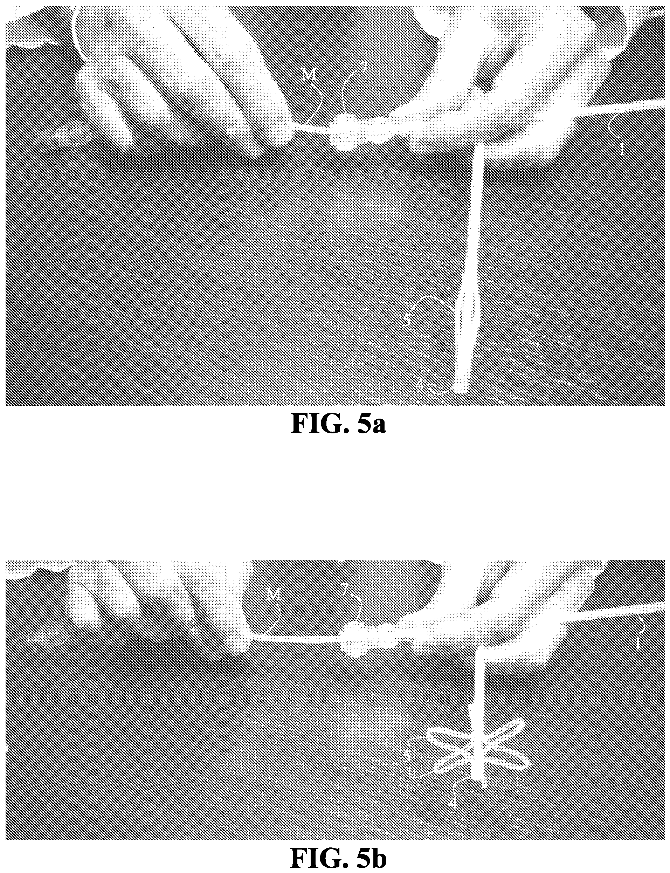

Generally, a device for inserting a guidewire suitable, for example, for heart valve prostheses in a blood vessel, comprises an external catheter 1 having a wall defining a longitudinal axis and a circumferential direction, and an internal catheter 2 inserted into the external catheter 1, having a handle portion M, shown in Figures 5a and 5b, configured to allow a user to advance or retract the internal catheter 2 along the longitudinal axis. The external catheter 1 defines an anchoring element to the internal walls of a blood vessel and comprises:
- a distal annular portion 4 of the wall of the external catheter 1, fixed to the internal catheter 2 so as to move integrally with the internal catheter 2 when it is advanced or retracted,
- a proximal annular portion 3 of the wall of the external catheter 1, sliding on the internal catheter 2, wherein the wall of the external catheter 1 does not have cuts in correspondence with the distal annular portion 4 and with the proximal annular portion 3,
- a median annular portion of the wall of the external catheter 1, sliding on the internal catheter 2, comprising at least a first plurality of notches directed according to the longitudinal axis and having a first length, a second plurality of notches directed according to the longitudinal axis and having a second length, in which all the notches divide the wall of the median annular portion of the external catheter 1 and define between them a plurality of threadlike elements 5 suspended above the internal catheter 2.

The threadlike elements 5 allow a self-centering / spacing of the device to the internal walls of a blood vessel because they are configured for:
- folding away radially with respect to the internal catheter 2 when the handle portion M is pulled, causing the internal catheter 2 to retract along the longitudinal axis with respect to the external catheter 1, as shown in figure 5b, abutting against the internal walls of the blood vessel in opposite points, counteracting displacements of the internal catheter 2 transversely to the longitudinal axis,
- extending along the longitudinal axis when the handle portion M is pushed by advancing the internal catheter 2 along the longitudinal axis with respect to the external catheter 1.

A characteristic of the device is therefore the fact that it allows the thread-like elements 5 to be folded / stretched through the internal catheter 2 with respect to the external catheter 1 by dragging with it the distal annular portion 4 of the external catheter 1, allowing them to abut / space themselves as desired against the inner walls of the blood vessel.

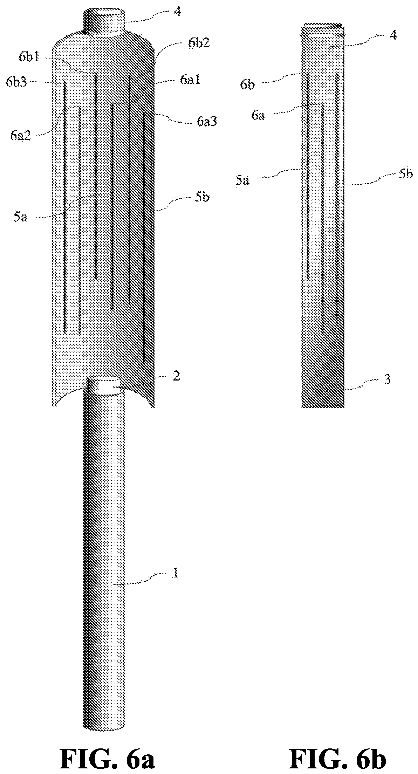

In the device of the present disclosure, shown in the sectional views of figures 6a and 6b and in the perspecive view of figures from 7a to 7c, the lengths of the notches of the first plurality of notches are different from the lengths of the notches of the second plurality of notches, and the notches of the first plurality of notches have respective ends 6a misaligned along the circumferential direction to respective ends 6b of the notches of the second plurality of notches, so that the notches of the first plurality of notches are offset along the circumferential direction with respect to the notches of the second plurality of notches.

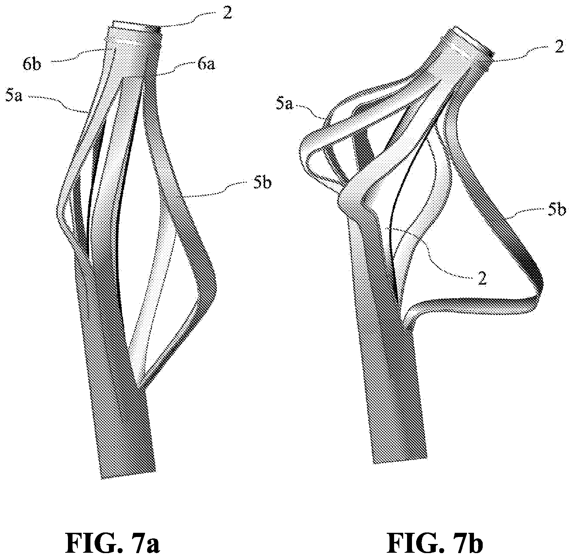

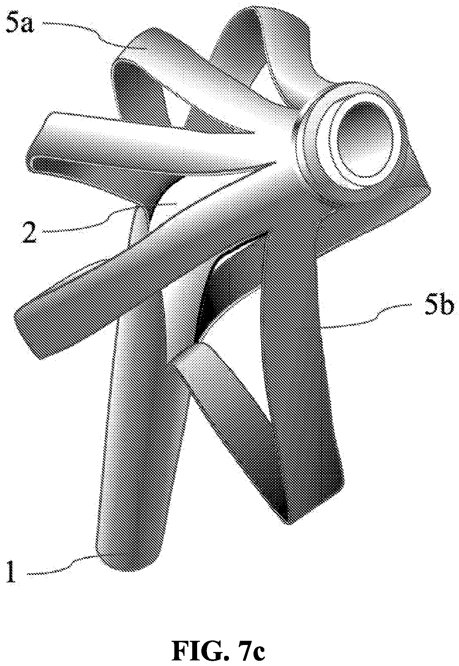

According to one aspect, shown in the sectional views of Figures 6a and 6b, the notches 6a1, 6a2, 6a3 of the first plurality of notches have different lengths, and so also the notches 6b 1, 6b2 and 6b3 of the second plurality of notches. In the example of the figures, each notch of the first plurality of notches may have one of three possible nominal lengths while each notch of the second plurality of notches may have one of three other different possible nominal lengths. In general, each notch of the first plurality of notches has a respective length selected from a first plurality of nominal lengths and each notch of the second plurality of notches has a respective length selected from a second plurality of nominal lengths. In this way, it is possible to have filiform elements of different lengths arranged in various ways along the circumferential direction. When the internal catheter 2 is retracted along the longitudinal axis with respect to the external catheter 1, the thread-like elements of different length will flex differently, as shown in the perspective views of Figures 7a to 7c, so that the orientation of the distal opening of the internal catheter 2 can be curved at will. Thanks to this technique, it is possible to bend in a more or less accentuated way the direction along which a guide wire that emerges from the internal catheter 2 will be directed: when the thread-like elements are completely extended, the internal catheter 2 is straight and a guide inserted in it will come out along a first direction coinciding with the longitudinal rectilinear axis of the catheter 2; when the internal catheter 2 is retracted, as shown in figure 7c, a guide wire will be oriented in a second direction more or less inclined than the first direction when it comes out of the distal opening of the catheter 2. By adjusting the force with which it is pulled the internal catheter 2, the surgeon can vary at will the direction along which the guide wire will come out and thus can orient it so as to pass through a heart valve on which to operate.

The notches of the first plurality of notches are orderly alternated with the notches of the second plurality of notches along the circumferential direction of the median portion of the wall of the external catheter 1, as shown in figures from 3 to 7c, so that each threadlike element 5 is defined by a notch of the first plurality and by a notch of the second plurality.

At least a first threadlike element of the threadlike elements is shorter than all the other threadlike elements and at least a second threadlike element is longer than all the other threadlike elements and is diametrically opposite to the first threadlike element.

According to one optional aspect, as shown in figures 3 and 4, the device may be equipped with a hemostatic valve 7 fixed to the external catheter 1, configured so that the internal catheter 2 slides tightly therethrough along the longitudinal axis. According to one aspect, the device of the present disclosure may comprise also a guiding catheter (not shown), preferably preformed, slidably inserted in said internal catheter 2, so as to further direct the guide wire into a blood vessel in proximity of a heart valve.

According to one aspect, the threadlike elements 5 are made of a biocompatible material suitable to be put into contact with human tissues.

According to an aspect of the present disclosure, the thread-like elements 5 can very simply be made by making the first plurality of notches and the second plurality of notches on a common catheter for surgical operations. For example, these notches can be made by machine using suitable blades, or they can be defined by laser cutting. According to an aspect of the present disclosure, the distal portion 4, to which the thread-like elements 5 are connected, can be fixed to the internal catheter 2 by welding or gluing or other valid method of mechanical joining.

The present invention has been described so far with reference to preferred embodiments. It is understood that there could be other embodiments which refer to the same inventive concept defined by the scope of the following claims.

## Claims

1. A device for inserting a guide wire suitable for heart valve prostheses into a blood vessel, comprising:
an external catheter (1) having a wall defining a longitudinal axis and a circumferential direction,
an internal catheter (2) inserted into said external catheter (1), having a handle portion (M) configured to allow a user to advance or retract said internal catheter (2) along said longitudinal axis,
wherein said external catheter (1) defines an anchoring element to the internal walls of a blood vessel,
said anchoring element comprising:
- a distal annular portion (4) of said wall of the external catheter (1), fixed to said internal catheter (2) so as to move integrally with said internal catheter (2) when it is advanced or retracted,
- a proximal annular portion (3) of said wall of the external catheter (1), sliding on said internal catheter (2), wherein the wall of the external catheter (1) does not have cuts in correspondence with said distal annular portion (4) and with said proximal annular portion (3),
- a median annular portion of said wall of the external catheter (1), sliding on said internal catheter (2), comprising at least a first plurality of notches directed according to said longitudinal axis, a second plurality of notches directed according to said longitudinal axis, in which all said notches divide the wall of the median annular portion of the external catheter (1) and define between them a plurality of threadlike elements (5) suspended above said internal catheter (2);
wherein said threadlike elements (5) are configured for:
- folding away radially with respect to said internal catheter (2) when said handle portion (M) is pulled, causing the internal catheter (2) to retract along said longitudinal axis with respect to the external catheter (1), abutting against said internal walls of the blood vessel in opposite points, counteracting displacements of the internal catheter (2) transversely to said longitudinal axis,
- extending along the longitudinal axis when said handle portion (M) is pushed by advancing the internal catheter (2) along said longitudinal axis with respect to the external catheter (1);
wherein each notch of the first plurality of notches has a respective length selected from a first plurality of nominal lengths, each notch of the second plurality of notches has a respective length selected from a second plurality of nominal lengths, and said notches of the first plurality of notches have respective ends (6a) misaligned along said circumferential direction to respective ends (6b) of the notches of the second plurality of notches, so that the notches of the first plurality of notches are offset along the circumferential direction with respect to the notches of the second plurality of notches;
**characterized in that** the notches of said first plurality of notches are orderly alternated with the notches of said second plurality of notches along said circumferential direction of the median portion of the wall of the external catheter (1), so that each threadlike element of said threadlike elements (5) is defined by a notch of the first plurality of notches and by a notch of the second plurality of notches, and
at least a first threadlike element of said threadlike elements (5) is shorter than all the other threadlike elements (5) and that at least a second threadlike element is longer than all the other threadlike elements (5) and is diametrically opposite to said first threadlike element.

2. The device according to the preceding claim, in which the notches of said first plurality of notches have their respective ends (6a) aligned with each other along said circumferential direction of the median portion of the wall of the external catheter (1), and the notches of said second plurality of notches have their respective ends (6b) aligned with each other along said circumferential direction of the median portion of the wall of the external catheter (1).

3. The device according to one of the preceding claims, comprising a guide wire for heart valve prostheses inserted in said internal catheter (2).

4. The device according to one of the preceding claims, comprising a preformed guiding catheter slidably inserted in said internal catheter (2).

5. The device according to one of the preceding claims, in which said threadlike elements are made of a biocompatible material.

6. The device according to one of the preceding claims, further comprising a hemostatic valve (7) fixed to said external catheter (1), configured so that said internal catheter (2) slides tightly therethrough along said longitudinal axis.

## Patentansprüche

1. Vorrichtung zum Einführen eines Führungsdrahtes, der für Herzklappenprothesen geeignet ist, in ein Blutgefäß, umfassend:
einen Außenkatheter (1), der eine Wand aufweist, die eine Längsachse und eine Umfangsrichtung definiert,
einen Innenkatheter (2), der in den Außenkatheter (1) eingeführt ist, der einen Griffabschnitt (M) aufweist, der konfiguriert ist, um einem Benutzer zu ermöglichen, den Innenkatheter (2) entlang der Längsachse vorzuschieben oder zurückzuziehen,
wobei der Außenkatheter (1) ein Verankerungselement an den Innenwänden eines Blutgefäßes definiert,
das Verankerungselement umfassend:
- einen distalen ringförmigen Abschnitt (4) der Wand des Außenkatheters (1), der an dem Innenkatheter (2) befestigt ist, um sich mit dem Innenkatheter (2) einstückig zu bewegen, wenn er vorgeschoben oder zurückgezogen wird,
- einen proximalen ringförmigen Abschnitt (3) der Wand des Außenkatheters (1), der sich auf dem Innenkatheter (2) verschiebt, wobei die Wand des Außenkatheters (1) keine Einschnitte in Übereinstimmung dem distalen ringförmigen Abschnitt (4) und dem proximalen ringförmigen Abschnitt (3) aufweist,
- einen mittleren ringförmigen Abschnitt der Wand des Außenkatheters (1), der sich auf dem Innenkatheter (2) verschiebt, umfassend mindestens eine erste Vielzahl von Kerben, die gemäß der Längsachse gerichtet sind, eine zweite Vielzahl von Kerben, die gemäß der Längsachse gerichtet sind, wobei alle Kerben die Wand des mittleren ringförmigen Abschnitts des Außenkatheters (1) unterteilen und zwischen sich eine Vielzahl von fadenförmigen Elementen (5) definieren, die über dem Innenkatheter (2) aufgehängt sind;
wobei die fadenförmigen Elemente (5) konfiguriert sind zum:
- radialen Wegklappen in Bezug auf den Innenkatheter (2), wenn der Griffabschnitt (M) gezogen wird, wodurch veranlasst wird, dass sich der Innenkatheter (2) entlang der Längsachse in Bezug auf den Außenkatheter (1) zurückzieht, Anliegen gegen die Innenwände des Blutgefäßes an entgegengesetzten Punkten, Entgegenwirken von Verlagerungen des Innenkatheters (2) quer zu der Längsachse,
- Erstrecken entlang der Längsachse, wenn der Griffabschnitt (M) durch Vorschieben des Innenkatheters (2) entlang der Längsachse in Bezug auf den Außenkatheter (1) geschoben wird;
wobei jede Kerbe der ersten Vielzahl von Kerben eine jeweilige Länge aufweist, die aus einer ersten Vielzahl von Nennlängen ausgewählt ist, jede Kerbe der zweiten Vielzahl von Kerben eine jeweilige Länge aufweist, die aus einer zweiten Vielzahl von Nennlängen ausgewählt ist, und die Kerben der ersten Vielzahl von Kerben jeweilige Enden (6a) aufweisen, die entlang der Umfangsrichtung zu jeweiligen Enden (6b) der Kerben der zweiten Vielzahl von Kerben falsch ausgerichtet sind, so dass die Kerben der ersten Vielzahl von Kerben entlang der Umfangsrichtung in Bezug auf die Kerben der zweiten Vielzahl von Kerben versetzt sind; **dadurch gekennzeichnet, dass** die Kerben der ersten Vielzahl von Kerben sich mit den Kerben der zweiten Vielzahl von Kerben entlang der Umfangsrichtung des Mittelabschnitts der Wand des Außenkatheters (1) ordnungsgemäß abwechseln, so dass jedes fadenförmige Element der fadenförmigen Elemente (5) durch eine Kerbe der ersten Vielzahl von Kerben und durch eine Kerbe der zweiten Vielzahl von Kerben definiert ist, und
mindestens ein erstes fadenförmiges Element der fadenförmigen Elemente (5) kürzer als alle anderen fadenförmigen Elemente (5) ist und dass mindestens ein zweites fadenförmiges Element länger als alle anderen fadenförmigen Elemente (5) ist und dem ersten fadenförmigen Element diametral entgegengesetzt angeordnet ist.

2. Vorrichtung nach einem der vorstehenden Ansprüche, bei der die jeweiligen Enden (6a) der Kerben der ersten Vielzahl von Kerben entlang der Umfangsrichtung des Mittelabschnitts der Wand des Außenkatheters (1) aufeinander ausgerichtet sind und die jeweiligen Enden (6b) der Kerben der zweiten Vielzahl von Kerben entlang der Umfangsrichtung des Mittelabschnitts der Wand des Außenkatheters (1) aufeinander ausgerichtet sind.

3. Vorrichtung nach einem der vorstehenden Ansprüche, umfassend einen Führungsdraht für Herzklappenprothesen, der in den Innenkatheter (2) eingeführt ist.

4. Vorrichtung nach einem der vorstehenden Ansprüche, umfassend einen vorgeformten Führungskatheter, der in den Innenkatheter (2) verschiebbar eingeführt ist.

5. Vorrichtung nach einem der vorstehenden Ansprüche, wobei die fadenförmigen Elemente aus einem biokompatiblen Material hergestellt sind.

6. Vorrichtung nach einem der vorstehenden Ansprüche, ferner umfassend ein an dem Außenkatheter (1) befestigtes hämostatisches Ventil (7), das so konfiguriert ist, dass sich der Innenkatheter (2) entlang der Längsachse dicht dahindurch verschiebt.

## Revendications

1. Dispositif permettant d'insérer un fil guide approprié pour des prothèses de valves cardiaques dans un vaisseau sanguin, comprenant :
un cathéter externe (1) ayant une paroi définissant un axe longitudinal et une direction circonférentielle,
un cathéter interne (2) inséré dans ledit cathéter externe (1), ayant une partie de poignée (M) conçue pour permettre à un utilisateur d'avancer ou de rétracter ledit cathéter interne (2) le long dudit axe longitudinal,
dans lequel ledit cathéter externe (1) définit un élément d'ancrage aux parois internes d'un vaisseau sanguin,
ledit élément d'ancrage comprenant :
- une partie annulaire distale (4) de ladite paroi du cathéter externe (1), fixée audit cathéter interne (2) de façon à se déplacer d'un seul tenant avec ledit cathéter interne (2) lorsqu'il est avancé ou rétracté,
- une partie annulaire proximale (3) de ladite paroi du cathéter externe (1), pouvant coulisser sur ledit cathéter interne (2), dans lequel la paroi du cathéter externe (1) n'a aucune interruption en correspondance avec ladite partie annulaire distale (4) et avec ladite partie annulaire proximale (3),
- une partie annulaire médiane de ladite paroi du cathéter externe (1), pouvant coulisser sur ledit cathéter interne (2), comprenant au moins une première pluralité d'encoches dirigées selon ledit axe longitudinal, une seconde pluralité d'encoches dirigées selon ledit axe longitudinal, dans lequel toutes lesdites encoches divisent la paroi de la partie annulaire médiane du cathéter externe (1) et définissent entre elles une pluralité d'éléments filiformes (5) suspendus au-dessus dudit cathéter interne (2) ;
dans lequel lesdits éléments filiformes (5) sont conçus pour :
- se replier de façon radiale par rapport audit cathéter interne (2) lorsque ladite partie de poignée (M) est tirée, faisant en sorte que le cathéter interne (2) se rétracte le long dudit axe longitudinal par rapport au cathéter externe (1), venant en butée contre lesdites parois internes du vaisseau sanguin en des points à l'opposé, contrebalançant les déplacements du cathéter interne (2) transversalement audit axe longitudinal,
- s'étendre le long de l'axe longitudinal lorsque ladite partie de poignée (M) est poussée en faisant avancer le cathéter interne (2) le long dudit axe longitudinal par rapport au cathéter externe (1) ;
dans lequel chaque encoche de la première pluralité d'encoches a une longueur respective choisie parmi une première pluralité de longueurs nominales, chaque encoche de la seconde pluralité d'encoches a une longueur respective choisie parmi une seconde pluralité de longueurs nominales, et lesdites encoches de la première pluralité d'encoches ont des extrémités respectives (6a) désalignées le long de la direction circonférentielle par rapport aux extrémités respectives (6b) des encoches de la seconde pluralité d'encoches, de sorte que les encoches de la première pluralité d'encoches sont décalées le long de la direction circonférentielle par rapport aux encoches de la seconde pluralité d'encoches ; **caractérisé en ce que** les encoches de ladite première pluralité d'encoches sont alternées de manière ordonnée avec les encoches de ladite seconde pluralité d'encoches le long de ladite direction circonférentielle de la partie médiane de la paroi du cathéter externe (1), de sorte que chaque élément filiforme desdits éléments filiformes (5) est défini par une encoche de la première pluralité d'encoches et par une encoche de la seconde pluralité d'encoches, et
au moins un premier élément filiforme desdits éléments filiformes (5) est plus court que tous les autres éléments filiformes (5) et au moins un second élément filiforme est plus long que tous les autres éléments filiformes (5) et est diamétralement opposé audit premier élément filiforme.

2. Dispositif selon l'une quelconque des revendications précédentes, dans lequl les encoches de ladite première pluralité d'encoches ont leurs extrémités respectives (6a) alignées les unes avec les autres le long de ladite direction circonférentielle de la partie médiane de la paroi du cathéter externe (1), et les encoches de ladite seconde pluralité d'encoches ont leurs extrémités respectives (6b) alignées les unes avec les autres le long de ladite direction circonférentielle de la partie médiane de la paroi du cathéter externe (1).

3. Dispositif selon l'une quelconque des revendications précédentes, comprenant un fil guide pour des prothèses de valves cardiaques inséré dans ledit cathéter interne (2).

4. Dispositif selon l'une quelconque des revendications précédentes, comprenant un cathéter de guidage préformé inséré de manière coulissante dans ledit cathéter interne (2).

5. Dispositif selon l'une quelconque des revendications précédentes, dans lequel lesdits éléments filiformes sont constitués d'un matériau biocompatible.

6. Dispositif selon l'une quelconque des revendications précédentes, comprenant en outre une valve hémostatique (7) fixée audit cathéter externe (1), conçue de sorte que ledit cathéter interne (2) glisse fermement à travers celle-ci le long dudit axe longitudinal.
